# EUROPEAN PATENT APPLICATION

(11) **EP 1 277 481 A1**
(43) Date of publication of application: **22.01.2003**
(21) Application number: 01117186.5
(22) Date of filing: 16.07.2001
(51) Int. Cl.: A61L 9/12, A61L 9/04

(54) **Gel air fresheners**

(71) Applicant: Givaudan SA, 1214 Vernier (CH)
(72) Inventor: Brown, Colin, Engham, Surrey, TW20 8LP (GB); Anderson, Simone, Ashford, Middlesex, TW15 2AJ (GB); Hart, Gerald Lieslie, Surbiton, Surrey, KT5 8BD (GB); Naish, Guy, Bicester, Oxfordshire, OX26 4US (GB)
(74) Representative: Simmons, John Murray, Dr.

(57) **Abstract**

A gel air-freshener device comprising a container defining a cavity for receiving a fragrance-containing gel matrix (7), the container having an aperture (4) for permitting ingress of air into the cavity and egress of fragrance from the cavity, and having first (1) and second ends (3), which ends abut the gel-matrix (7) and move freely relative to each other as the gel matrix (7) shrinks upon evaporation of the fragrance. The device exploits the shrinkage of the gel-matrix (7) and translates this into movement of the device thereby providing a visual cue to the condition of the gel matrix and in particular when it is exhausted.

## Description

This invention is concerned with air freshener devices, and in particular gel air freshener devices incorporating a fragrance within a gel matrix.

Air freshener devices known in the art may consist of a hollow container defining an internal cavity for receiving a fragrance-containing material. The container walls are provided with apertures that permit circulation of air throughout the internal cavity to encourage release of the fragrance which may be released to the ambient air by means of the apertures.

Other air-freshener devices are known wherein the container comprises two connected parts that are adapted to move relative to each other in a telescoping fashion. At least one of the connected parts is provided with apertures for permitting air to circulate throughout the container. By sliding the telescoping parts together or apart in order to open or close apertures one can control the flow of air through the container. The connected parts are not free-moving with respect to each other however, and movement is only achieved manually by the user.

Devices of the type referred to above contain a fragrance that is contained on or within a suitable support material, for example a suitably absorbent material. Popular support materials are gels which can be formed into self-supporting matrices containing a fragrance. However, a feature of such gel matrices is that they shrink as the fragrance and other volatiles they contain are released by evaporation. This feature makes the exhausted matrices easy to dispose of after use but it is also associated with certain disadvantages, for example the reduction in their surface area and the concomitant depletion of fragrance concentration during use means that gel matrices become increasingly less efficient at dispensing fragrance as they age. Accordingly, matrices need to be replaced at regular intervals if efficient air-freshening activity is to be maintained. If a matrix is replaced too soon then this represents an inefficient use of the matrix; whereas if it is replaced at too infrequent intervals the air-freshening properties will be compromised.

A user could place a device close to his nose and smell it to determine whether fragrance is still being released, but due to the very low detection threshold of many fragrances, smelling fragrance in this way is not a satisfactory indicator that the air-freshener is functioning correctly. Unfortunately therefore, it remains the case that current air-freshener devices do not provide the user with reliable means for determining the condition of gel matrices and in particular whether they are exhausted.

Surprisingly however, it has now been found that it is possible to make a gel air freshener device which is substantially free from the problems associated with the prior art.

The invention therefore provides a gel air-freshener device comprising a container defining a cavity for receiving a fragrance-containing gel matrix, the container having an aperture for permitting ingress of air into the cavity and egress of fragrance from the cavity, and having first and second ends, which ends abut the gel-matrix and move freely relative to each other as the gel matrix shrinks upon evaporation of the fragrance.

The device is quite unlike any prior art air-freshener device in that it exploits the shrinkage of a gel-matrix to permit the free-moving ends of the device to move relative to each other and thereby act as a visual cue as to the condition of the gel matrix, and in particular to provide a visual cue when it is exhausted. By the phrase "free-moving" is meant that at least one of the ends move in conformity with the shrinkage of the gel-matrix and require no urging by a user.

In a first embodiment of the invention there is provided a gel air freshener device comprising a container having first and second segments, each segment comprising side walls, an end wall and an open end, at least one of the segments additionally comprising apertures in the side walls, one open end receives the other open end such that the segments define a cavity for receiving therein a fragrance-containing gel matrix, which gel matrix abuts each end wall, wherein the segments are free-moving in a telescoping manner with respect to each other as the gel matrix shrinks upon evaporation of the fragrance.

The device has numerous advantages: The combination of segments that are free-moving with respect to each other, and the abutment relationship of the gel matrix with each end wall provides that the segments are held apart at their maximum extension when a new gel matrix is placed into the device, and as the gel matrix shrinks as the fragrance evaporates so the segments of the device move together longitudinally in a telescoping manner in conformity with the shrinking gel matrix. The user is thereby provided with a visual cue as to the condition of the air freshener device. This device is therefore quite unlike the prior art devices employing containers having telescopically moveable segments, which prior art devices comprise segments having side walls that are in such frictional engagement with each other, for example by being resiliently biased against each other, that frictional force holds the segments static with respect to each other until a user forcibly pushes them together or pulls them apart.

The movement of one segment relative to the other provides a visual cue as to the condition of the gel matrix. The visual end-point, indicative of the gel matrix being exhausted, may be provided by the spatial relationship of the segments, e.g. the gel matrix being taken as exhausted when the second segment is withdrawn inside the first segment. Alternatively, other, or additional, visual cues may be provided, for example markings may be provided on the external surfaces of the device, the disappearance or appearance of which as the segments move relative to each other indicating the condition of the gel matrix. Markings may be provided that are calibrated with the shrinkage of a particular type of gel matrix such that the user is given an indication, e.g. a quantitative indication, as to how many days use a gel matrix has remaining of useful operation. The visual markings may be provided by the apertures on the container walls such that the exhaustion of the matrix is indicated by the closure of the apertures.

Other visual indicators are contemplated. For example, in a variation of this first embodiment, a shaft located internally of the device and fixed to one end wall, may extend through the gel matrix provided with a passage for this purpose, and through the other end wall by means of a suitably positioned aperture in the other end wall, as one segment moves relative to the other. The shaft would appear progressively more prominently as the gel matrix shrinks and the segments close together. Additionally, the shaft may contain an additional marking that indicates exhaustion of the gel matrix. Further visual indicators, that exploit the relative movement of the segments are contemplated by the present invention as will be obvious to the skilled addressee.

The movement of one segment relative to another is most simply achieved by means of gravity which acts on the device and is prevented from pushing the segments together by the intervention of the gel matrix abutting the end walls. However, as the fragrance evaporates and the gel matrix shrinks, so gravity will urge the end walls of each segment to remain in contact with the gel matrix and thereby push the segments together. Such an arrangement is preferable for ease of design of the device and will work, for example when the segments are intended to move longitudinally in a telescoping manner in the direction of gravitational force, e.g. as in the case of an air-freshener device intended to be a free-standing device. However, if it is intended that the segments of the device should move longitudinally with respect to each other in a direction other than the direction of gravitational force, one may uses other means to urge the segments together as the gel matrix shrinks. For example, movement of the segments may be achieved by use of springs or other resilient means that act to urge the segments together.

The container may be generally of cylindrical shape. The first segment therefore may have the form of an open ended cylinder. The second segment may also have the form of an open-ended cylinder. One segment may have a slightly larger diameter than the other. For example, the first segment may be of slightly larger diameter than the second segment in which case the open end of the first segment receives the first segment and the second segment slides freely within the first segment. Whereas the container may be generally described in terms of its cylindrical shape, the skilled person will appreciate that the device may be constructed in other geometrical shapes provided they permit of free-movement of the segments. The device may in addition to its basic geometrical form include other fanciful or aesthetic design elements without departing from the general inventive concept.

Optionally, towards their open ends each of the first and second segments is provided with a circumferential lip. The lips are opposed such that when the segments are moved longitudinally apart, the opposed lips will abut each other preventing the separation of the two segments.

Whereas the device of the first embodiment has been described with reference to only two co-operating segments which have end walls, a modification of this embodiment is contemplated wherein device may comprise a third segment, for example in the form of an open ended cylinder, interposed between said first and second segments and which has a diameter intermediate between first and second segments such that it may be received by the first segment and freely move within the first segment; and such that it may receive the second segment which may be free-moving within the third segment. The third segment is also optionally provided with circumferential lips towards each open end, preventing detachment from the first and second segments from the third segment. The skilled person will understand that still further segments could be incorporated into a device according to the invention in a manner described above without departing from the general inventive concept.

As referred to above, at least one of the walls of the segments is provided with apertures which communicate with the cavity to allow air to flow through the cavity and to allow the fragrance to egress from the device. The apertures may be of any shape, e.g. discrete annular apertures or elongate openings, and they may arranged in a decorative fashion, for example to give the device the appearance of a decorative cage encasing the gel matrix.

One segment, e.g. the first segment may act as a base which may stand on a surface or may be fixed to a wall with suitable fixing means, e.g. a bracket. In this arrangement the first segment remains static and the second segment is free-moving relative to the first segment.

The device may be a use-once device that is intended for disposal after the gel-matrix is exhausted. However, for economical reasons, it is preferable if the device is adapted for receiving refill gel-matrices. Preferably therefore, the device is provided with means for inserting a gel-matrix into the cavity and means for removing it when it is exhausted. These means may be provided simply be separating the segments and inserting or removing a matrix via the open end of a segment and thereafter reassembling the segments. However, in devices wherein the circumferential lips do not permit the segments to separate (at least not easily),an end wall of a segment may be removable to permit introduction or removal of a gel matrix. The end wall may be completely detachable or it may be openable and remain attached to the segment by means of a hinge.

Upon insertion of a refill gel matrix into the device, the gel matrix is forced against one end wall and thereafter the device is reassembled such that the matrix abuts both end walls and drives the telescoping segments apart and urges the device into its fully extended position.

For ease of handling gel matrix refills, they may be supplied already pre-cast in a segment of the device. Thus, for example, the refill may comprise a gel matrix pre-cast in the second segment such that the gel is essentially flush with the open end of the second segment and a foil seal may be provided to protect the gel matrix prior to use. In a manner described above, the end wall of the first segment may be detached to permit access of the second segment.

However, in this variation of the first embodiment, the internal surface of the end wall of the first segment is provided with a raised platform such that when the end wall is reattached to the first segment, the raised platform abuts the gel-matrix that is flush with the open end of the second segment and drives the second segment upwards through the open end of the first segment whereupon the second segment is driven apart from the first segment in a telescoping fashion to its maximum extension. Optionally, circumferential lips prevent the second segment from detaching from the first segment during this procedure. As the gel matrix shrinks, the second segment moves relative to the first segment in the direction of the first segment and a visual cue of the exhaustion of the gel matrix may be indicated, for example by the disappearance of the second segment within the first segment. Movement of the second segment relative to the first segment is not hindered by the raised platform as the raised platform is of smaller dimension than the second segment which can collapse over the platform without obstruction.

In a second embodiment of the invention the gel air freshener device may comprise a coil of unitary construction which, when extended has the general appearance of a pyramidal coil. The coil when extended defines a cavity for receiving a fragrance-containing gel matrix. The coil is held in its extended configuration by the matrix and is resilient and acts like a spring, retracting as the gel matrix shrinks. One end of the coil may form a base upon which the device may stand when in a free-standing arrangement; the other end of the coil terminates in a tip which defines the top of the pyramid when the device is in extended form. Because of the resilient nature of the coil, the tip will be drawn in the direction of the base as the gel-matrix shrinks.

Devices according to the present invention may be formed of any rigid material that is self-supporting and which can be easily formed into complex shapes, for example by moulding or extruding. Materials that may be mentioned are plastics materials, metals, ceramics or glass. Compressed paper could also be used, however, given the proximity of the gel-matrix to the device, to prevent staining of the device the paper would have to be coated or otherwise treated to render it stain-resistant.

In another aspect of the present invention there is provided a fragrance-containing gel matrix for use in an air-freshener device as hereinabove described.

A gel matrix may be formed from any gelling composition that can be moulded into suitable shapes and that, when set, is self supporting and is capable substantially resisting mechanical deformation as a result of pressure exerted by the end walls of a container.

Gels have long been know as release media for fragrances or other volatile materials and there are a wide range of gelling agents or materials known in the art that are appropriate to such an application and anyone versed in the art would be able to formulate a gel matrix for use in the present invention. One of the most common materials is carrageenan, a naturally-occurring family of carbohydrates extracted from red seaweed. A typical example of a commercially available carregeenan material is Danagel AF 9254 from FMC BioPolymer, United Kingdom. However, other types of gel may be employed for example, those based on starch, carboxymethyl cellulose and amide polyacrylamide polymers reacted with hypohalite salt solution.

For a device in which the aesthetics of a light source may be employed, it is preferred to use a clear gel. While carregeenan gels can be made semi-transparent, there are other materials that can be used to give a fully transparent media. These include gelatin, polysaccharides and other polymer systems more fully described in US5780527. This is not intended to be an exhaustive list of polymers however, and there are a wide number of materials that can be used as gelling agents which one versed in the art would be able to apply to such an application.

A gel-matrix of the present invention may contain from only 1 or 2% fragrance up to as much as ninety % or more.

It is a function of the gel matrix that it is subject to substantial shrinkage as a result of evaporation of fragrance and other volatile materials in order that this shrinkage is translated into movement of the device to provide the visual cue to the user. The ability of a gel matrix to shrink is related to the amount of solids contained in the gel. For substantial shrinkage to occur the solids content must be relatively low, without being so low as to compromise the ability for the matrix to be mechanically self supporting. In a preferred embodiment a gel-matrix contains as little as 2 to 10%, e.g. 2.5 to 5 %by weight solids, the remaining mass being volatiles, e.g. water and fragrance. In a preferred gel matrix composition, the dimensional shrinkage of a gel matrix, going from a fresh state to an exhausted state, in the direction of movement of the device may be greater than 50%, more particularly 55 to 65%, e.g. 63%.

A gel composition for use in forming a gel matrix may be made by methods generally known in the art. A specific method is disclosed in the Examples. A gel matrix formulation thus made may be poured into a mould to produce a gel matrix of any desired shape. A gel matrix will be configured to generally conform with the dimensions of the internal cavity defined by the gel-air freshener device, and must be dimensioned such that it abuts the end walls of the device when the device is in a fully extended position. In a preferred embodiment, the gel matrix may be provided pre-cast in a segment of the air-freshener device for ease of handling, and this segment is added to the air freshener device prior to use in a manner more fully described above.

A device according to the present invention will now be further described with reference to the drawings.

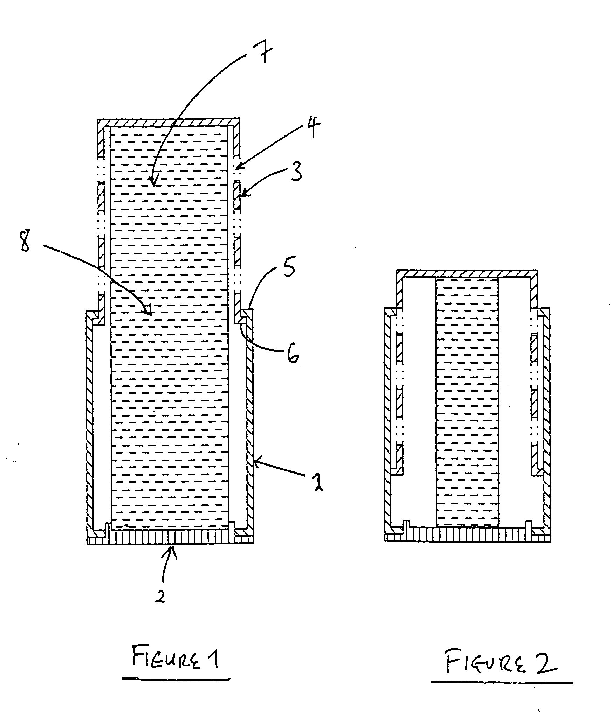

In Figure 1 there is shown in cross-section a gel air-freshener device comprising a container having a first segment (1) and a second segment (3). The first segment terminates at one end in a end wall (2) which in this embodiment may act as a base in a free-standing device. The end-wall (2) is removable in this embodiment to permit a gel matrix (7) to be inserted into the internal cavity defined by the first and second segments. The second segment is provided with a plurality of apertures (4) for permitting ingress of ambient air and egress of fragrance. At the end opposite the end wall (2) of the first segment, the first segment is provided with an opening (8). The second segment is of smaller diameter than the first segment and thus fits inside said first segment through this opening. The newly inserted gel matrix urges the segments apart and forces the device into its fully open position. The segments are prevented from separating by the opposing lip portions (5) and (6) which abut each other in the fully open position. The lips play another important role as they effectively reduce the area of each segment in frictional engagement with the other thereby reducing frictional resistance to the free movement of the segments. In use, the gel-matrix shrinks as fragrance and other volatiles evaporate and Figure 2 shows the same device in cross-section with the gel-matrix in an exhausted condition and the device in a closed position. Once exhausted, the gel-matrix may be removed through the removable end wall (2). The visual cue is enhanced in this embodiment, not only by the relative movement of the segments, but also by the disappearance of the apertures when the device is in its exhausted condition.

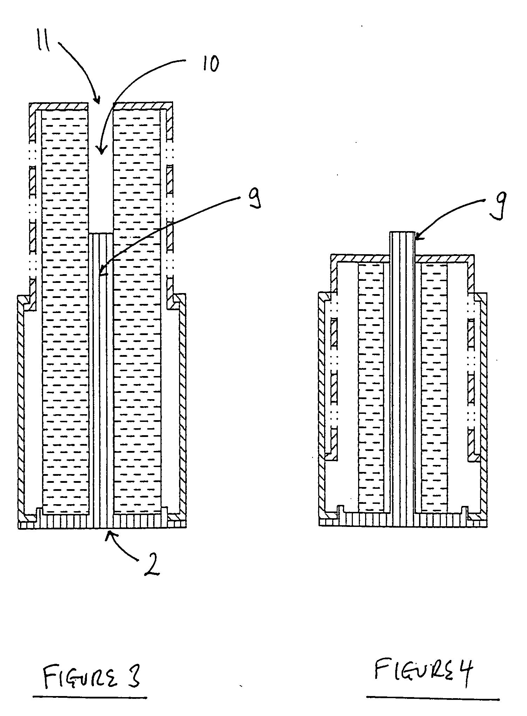

In Figures 3 and 4, a modification of the device of Figure 1 and Figure 2 is shown in cross-section. Thus, the modification consists of additional means of providing a visual cue consisting of a shaft (9) that extends from the inner surface of the end wall (2) and passes through the gel matrix by means of an elongate passage (10) provided therein which passage communicates with an opening (11) provided in the end wall of the second segment. As the gel matrix shrinks into its exhausted state so the relative movement of the segments causes the shaft to project from the opening (11) thereby providing an additional visual cue to the user that the gel matrix is exhausted.

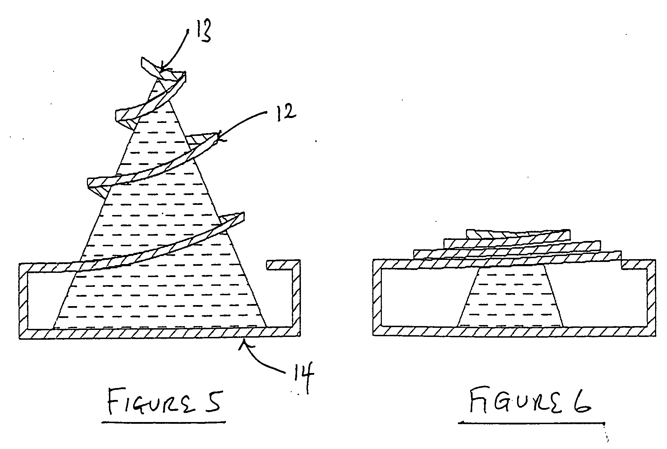

Figure 5 shows in side view a device in the form of a pyramidal-shaped coil (12). One end of the coil (13) abuts the gel matrix near the apex of its pyramidal form whereas the opposing end is in the form of a base (14) for supporting the gel matrix. In the view shown, the coil is in its extended form and the end abutting the gel matrix near its tip is resiliently biased towards the base such that the end of the coil abutting the gel matrix near its tip moves towards the base as the matrix shrinks. When the gel matrix is in its exhausted state the coil has collapsed on itself and provides a strong visual cue to the user (see Figure 6).

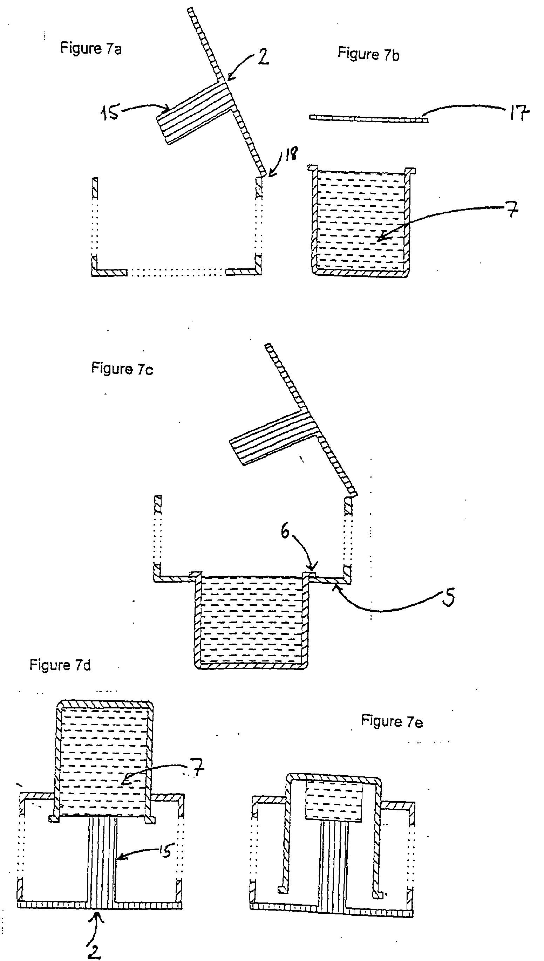

Figure 7 shows a variant of a gel air-freshener device shown in Figures 1 and 2 in cross-section. In particular Figure 7a shows a first segment of the device wherein the end wall (2) is open on a hinge (18) to receive the second segment (see Figure 7b). The inner surface of the end wall (2) is provided with a raised platform (15) that is provided to support the gel matrix (7). Figure 7b shows a second segment containing a pre-cast gel matrix (7). The gel matrix is protected prior to use with a removable foil (17) that covers the open end of the second segment (and is shown in this figure removed from the segment). Figure 7c shows a device wherein the foil (17) is removed and the second segment is inserted into the first segment such that it protrudes through the open end (8) of the first segment and is prevented from passing through the open end by the opposed circumferential lips (5,6). Figure 7d shows the complete air freshener device with the end wall closed and turned through 180 degrees into its operable arrangement with the end wall (2) acting as a base and the gel matrix (7) abutting the raised platform (15). Figure 7e shows the device of 7d with the gel matrix in an exhausted condition.

### Example

Example 1 describes a gel air freshener formulation based on carrageenan. In this example, the carrageenan used is Danagel AF 9254 from FMC BioPolymer, Unit 3C, Harcourt Way, Meridian Business Park, Leicester - LE3 2WP, United Kingdom.

Crillet 4 is the trade name for ethoxy (20) sorbitan monooleate from Croda Chemicals Ltd, Goole, North Humberside, United Kingdom.

| Material | Percent w/w |
|---|---|
| Carrageenan | 2.5 |
| Crillet 4 | 0.2 |
| Fragrance | 6.0 |
| Preservative | 0.1 |
| Water | 91.2 |

The gel is prepared using the following process method:

Three quarters of the water is heated to 50 degrees centigrade in a glass beaker. To this, the carrageenan is added slowly with vigorous stirring. After complete addition, the mixture is heated to 90 degrees centigrade for 30 minutes. The mixture is then removed from the heat source and the Crillet 4 added with stirring. The remaining water is then added and the temperature reduced to 70 degrees centigrade. The fragrance and preservative are then added and the mixture homogenised for 10 minutes at 63-68 degrees centigrade. The formula is then immediately poured into suitable moulds in which the gel is to be cast (i.e. containers from which the formed gel can be easily released) and allowed to cool and set.

The gels are cast into cylindrical and conical shapes and the dimensions measured (height). For both the cylinder and conical arrangements, height reductions are measured in the range of 55 to 63% over a seven day period.

## Claims

1. A gel air-freshener device comprising a container defining a cavity for receiving a fragrance-containing gel matrix, the container having an aperture for permitting ingress of air into the cavity and egress of fragrance from the cavity, and having first and second ends, which ends abut the gel-matrix and move freely relative to each other as the gel matrix shrinks upon evaporation of the fragrance.

2. A gel air freshener device according to claim 1 comprising a container having first and second segments, each segment comprising side walls, an end wall and an open end, the second segment additionally comprising apertures in the side walls, one open end receives the other open end such that the segments define a cavity for receiving therein a fragrance-containing gel matrix, which gel matrix abuts each end wall, wherein the segments are free-moving longitudinally with respect to each other as the gel matrix shrinks upon evaporation of the fragrance.

3. A device according to claim 2 wherein the movement of one segment relative to the other segment provides a visual cue to the user that the gel matrix is exhausted.

4. A device according to claim 3 wherein visual cues are provided by markers on the device that become visible or are rendered invisible as a result of the relative movement of the segments.

5. A device according to claim 3 or claim 4 wherein a visual cue is provided by the progressive disappearance of apertures provided in the side walls as a result of the relative movement of the segments.

6. A device according to any of the claims 2 to 5 comprising a third segment wherein the third segment is interposed between said first and second segments and which has dimensions intermediate between first and second segments such that it may be received by the first segment and freely move within the first segment in a telescoping manner; and such that it may receive the second segment which may be free-moving within the third segment in a telescoping manner.

7. A device according to any of the claims 2 to 6 wherein the segments move freely relative to each other by means of gravity.

8. A device according to claim 1 comprising a coil of unitary construction which, when extended has the general appearance of a pyramidal coil which defines a cavity internal of the coil for receiving a pyramid-shaped gel matrix, one end of the coil abuts the gel matrix towards its tip whereas the other end terminates in a base for supporting the base of the pyramid-shaped gel matrix, the end abutting the gel matrix near its tip is resiliently biased towards the base such that the end of the coil abutting the gel matrix near its tip moves towards the base as the matrix shrinks.

9. A gel matrix for use in a device as defined in any of the preceding claims.

10. A gel matrix according to claim 9 comprising 2 to 10 % by weight solids.

11. A gel matrix according to claim 9 or claim 10 wherein the height dimension decreases by up to 65% as the fragrance and other volatiles evaporate therefrom.

12. An gel air-freshener device substantially as hereinabove described with reference to the drawings.
